# EUROPEAN PATENT SPECIFICATION

(11) **EP 1 773 307 B1**
(45) Date of publication and mention of the grant of the patent: **15.10.2014**
(21) Application number: 05768530.7
(22) Date of filing: 29.07.2005
(51) Int. Cl.: A61K 31/133, A61K 47/10, A61P 37/00, A61P 37/06

(54) **COMPOUND FORMULATIONS OF 2-AMINO-1,3-PROPANEDIOL COMPOUNDS**
VERBINDUNGSFORMULIERUNGEN AUS 2-AMINO-1,3-PROPANEDIOL-VERBINDUNGEN
PREPARATIONS DE COMPOSES A BASE DE 2-AMINO-1,3-PROPANEDIOL

(30) Priority: 30.07.2004 US 592991 P
(43) Date of publication of application: 18.04.2007
(60) Divisional application: 14178519.6
(73) Proprietor: Novartis AG, 4056 Basel (CH); Novartis Pharma GmbH, 1230 Wien (AT)
(72) Inventor: BURANACHOKPAISAN, Thitiwan, Morristown, NJ 07960 (US); DANNENFELSER, Rose-Marie, Bloomfield, NJ 07003 (US); LI, Ping, Basking Ridge, NJ 07920 (US)
(74) Representative: Rouquayrol, Céline Hélène
(86) International application number: PCT/EP2005/008267
(87) International publication number: WO 2006/010630

(56) References cited:
- EP-A- 0 627 406
- EP-A- 0 812 588
- EP-A- 0 990 440
- EP-A- 1 050 301
- EP-A- 1 195 165
- WO-A-02/18395
- WO-A-03/062248
- WO-A-03/062252
- GB-A- 2 400 318

## Description

### Field of the Invention

This invention is concerned with pharmaceutical organic concentrate formulations of 2 amino-1,3-propanediol compounds, especially 2-amino-2-[2-(4-octylphenyl)ethyl]-propane-1,3-diol or a pharmaceutically acceptable salt thereof, at a concentration of 0.01-10 mg/ml in an organic solvent comprising 0-78.9 % (w/v) of ethanol in propylene glycol, and other invention embodiments in the claims which are incorporated here by reference. In particular, the invention is concerned with pharmaceutical formulations which are administrable parenterally, e.g., intravenously (i.v.).

### Background of the Invention

2-amino-1,3-propanediol compounds, in particular, 2-amino-2-[2-(4-octylphenyl)ethyl]-propane-1,3-diol (sometimes referred to as FTY720) as shown: 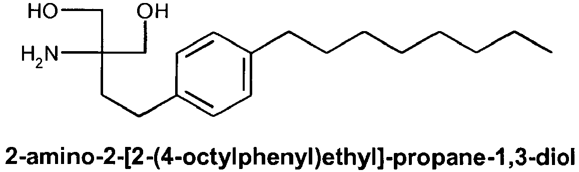 and a pharmaceutically acceptable salt thereof, are known to be useful as suppressants of rejection in organ or bone marrow transplantation or as therapeutic agents of various autoimmune diseases, as described, e.g., in EP 0627406B1.

While 2-amino-2-[2-(4-octylphenyl)ethyl]-propane-1,3-diol, particularly its salt form, is soluble in water, crystalline deposits of this compound have been observed in aqueous solutions either shortly after preparation or upon storage. Addition of cyclodextrins to aqueous solutions of the compound as described in U.S. Patent No. 6,476,004, have been found to be effective in reducing crystalline deposits of the compound, however, use of cyclodextrins has been limited by cost and regulation. Crystalline deposits of the compound have also been observed in aqueous solutions containing sodium laurylsulfate or polyvinylpyrrolidone K12 PF as described in the afore-mentioned U.S. Patent 6,476,004.

A semi-aqueous formulation of the compound which contains ethanol and polyethylene glycol has been described in the afore-mentioned EP 0627406B1. While there is no mention of the occurrence of crystalline deposits in this semi-aqueous formulation, the formulation exhibited problems such as local irritation and hemolysis upon i.v. injection due to the high concentration of ethanol and polyethylene glycol present in the formulation.

Accordingly, it still remains desirable, therefore, to develop physically stable pharmaceutical formulations of this compound and other 2-amino-1,3-propanediol compounds and pharmaceutically acceptable salts thereof, which are crystal-free upon storage, and such formulations as claimed are the subject matter of the present invention.

### Summary of the Invention

The present invention provides pharmaceutical concentrate formulations comprising 2 2-amino-2-[2-(4-octylphenyl)ethyl]-propane-1,3-diol or a pharmaceutically acceptable salt thereof, as claimed, which have improved stability, i.e., the formulations are free of crystals upon storage. The invention also provides pharmaceutical solutions of the compound as claimed which are formed by the addition of a diluent vehicle to the concentrate formulation prior to administration.

### Detailed Description of the Invention

The present disclosure in general for reference purposes relates to pharmaceutical organic concentrate formulations comprising 2-amino-1,3-propanediol compounds and salts thereof, particularly 2-amino-2-[2-(4-octylphenyl)ethyl]-propane-1,3-diol or a pharmaceutically acceptable salt thereof, and analogs of these compounds, in an organic solvent comprising ethanol and/or propylene glycol, and pharmaceutical semi-aqueous concentrate formulations comprising the afore-mentioned compounds in a semi-aqueous solvent comprising ethanol and propylene glycol. The concentrate formulations are free of crystals when stored at ambient and refrigerated conditions for extended periods of time, e.g., more than six months. The present disclosure for reference purposes also relates to pharmaceutical solutions which are formed by the addition of a diluent vehicle to the concentrate formulations prior to administration. The pharmaceutical solutions when administered parenterally are non-hemolytic, non-foaming and non-irritating.

Examples of the afore-mentioned compounds useful in the pharmaceutical concentrate formulations and pharmaceutical solutions of the present disclosure for reference purposes are compounds as disclosed in EP627406B1, e.g. a compound of formula I 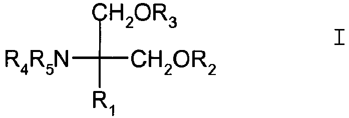 wherein R₁ is a straight- or branched (C₁₂₋₂₂)chain
- which may have in the chain a bond or a hetero atom selected from a double bond, a triple bond, O, S, NR₆, wherein R₆ is H, alkyl, aralkyl, acyl or alkoxycarbonyl, and carbonyl, and/or
   - which may have as a substituent alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, amino, hydroxyimino, hydroxy or carboxy; or
R₁ is
- a phenylalkyl wherein alkyl is a straight- or branched (C₆₋₂₀)carbon chain; or
- a phenylalkyl wherein alkyl is a straight- or branched (C₁₋₃₀)carbon chain wherein said phenylalkyl is substituted by
- a straight- or branched (C₆₋₂₀)carbon chain optionally substituted by halogen,
- a straight- or branched (C₆₋₂₀)alkoxy chain optionally substitued by halogen,
- a straight- or branched (C₆₋₂₀)alkenyloxy,
- phenylalkoxy, halophenylalkoxy, phenylalkoxyalkyl, phenoxyalkoxy or phenoxyalkyl,
- cycloalkylalkyl substituted by C₆₋₂₀alkyl,
- heteroarylalkyl substituted by C₆₋₂₀alkyl,
- heterocyclic C₆₋₂₀alkyl or
- heterocyclic alkyl substituted by C₂₋₂₀alkyl,
   and wherein
   the alkyl moiety may have
- in the carbon chain, a bond or a heteroatom selected from a double bond, a triple bond, O, S, sulfinyl, sulfonyl, or NR₆, wherein R₆ is as defined above, and
- as a substituent alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, amino, hydroxy or carboxy, and
   each of R₂, R₃, R₄ and R₅, independently, is H, C₁₋₄ alkyl or acyl
   or a pharmaceutically acceptable salt thereof;
- Compounds as disclosed in EP 1002792A1, e.g. a compound of formula II 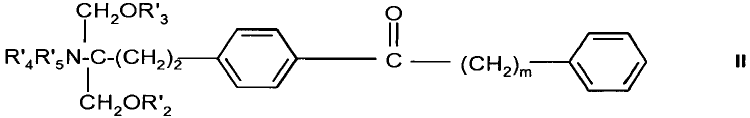 wherein m is 1 to 9 and each of R'₂, R'₃, R'₄ and R'₅, independently, is H, alkyl or acyl, or a pharmaceutically acceptable salt thereof;
- Reference compounds as disclosed in EP0778263 A1, e.g. a compound of formula III 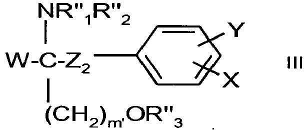 wherein W is H; C₁₋₆alkyl, C₂₋₆alkenyl or C₂₋₆alkynyl; unsubstituted or by OH substituted phenyl; R"₄O(CH₂)ₙ; or C₁₋₆alkyl substituted by 1 to 3 substituents selected from the group consisting of halogen, C₃₋₈cycloalkyl, phenyl and phenyl substituted by OH; X is H or unsubstituted or substituted straight chain alkyl having a number p of carbon atoms or unsubstituted or substituted straight chain alkoxy having a number (p-1) of carbon atoms, e.g. substituted by 1 to 3 substitutents selected from the group consisting of C₁₋₆ alkyl, OH, C₁₋₆alkoxy, acyloxy, amino, C₁₋₆alkylamino, acylamino, oxo, haloC₁₋₆alkyl, halogen, unsubstituted phenyl and phenyl substituted by 1 to 3 substituents selected from the group consisting of C₁₋₆alkyl, OH, C₁₋₆alkoxy, acyl, acyloxy, amino, C₁₋₆alkylamino, acylamino, haloC₁₋₆alkyl and halogen; Y is H, C₁₋₆alkyl, OH, C₁₋₆alkoxy, acyl, acyloxy, amino, C₁₋₆alkylamino, acylamino, haloC₁₋₆alkyl or halogen, Z₂ is a single bond or a straight chain alkylene having a number or carbon atoms of q,
   each of p and q, independently, is an integer of 1 to 20, with the proviso of 6≤p+q≤23, m' is 1, 2 or 3, n is 2 or 3,
   each of R"₁, R"₂, R"₃ and R"₄, independently, is H, C₁₋₄alkyl or acyl,
   or a pharmaceutically acceptable salt thereof,
- Reference compounds as disclosed in WO02/18395, e.g. a compound of formula IVa or IVb 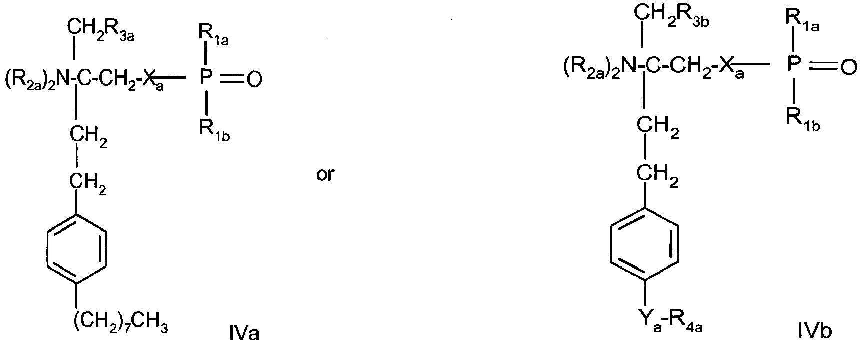 wherein Xₐ is O, S, NR₁ₛ or a group -(CH₂)ₙₐ-, which group is unsubstituted or substituted by 1 to 4 halogen; nₐ is 1 or 2, R₁ₛ is H or (C₁₋₄)alkyl, which alkyl is unsubstituted or substituted by halogen; R₁ₐ is H, OH, (C₁₋₄)alkyl or O(C₁₋₄)alkyl wherein alkyl is unsubstituted or substituted by 1 to 3 halogen; R_{1b} is H, OH or (C₁₋₄)alkyl, wherein alkyl is unsubstituted or substituted by halogen; each R₂ₐ is independently selected from H or (C₁₋₄)alkyl, which alkyl is unsubstituted or substitued by halogen; R₃ₐ is H, OH, halogen or O(C₁₋₄)alkyl wherein alkyl is unsubstituted or substituted by halogen; and R_{3b} is H, OH, halogen, (C₁₋₄)alkyl wherein
   alkyl is unsubstituted or substituted by hydroxy, or O(C₁₋₄)alkyl wherein alkyl is unsubstituted or substituted by halogen; Yₐ is -CH₂-, -C(O)-, -CH(OH)-, -C(=NOH)-, O or S, and R₄ₐ is (C₄₋₁₄)alkyl or (C₄₋₁₄)alkenyl;
   or a pharmaceutically acceptable salt or hydrate thereof;
- Reference compounds as disclosed in WO 02/076995, e.g. a compound of formula V 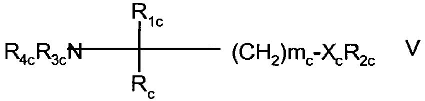 wherein
   - m_{c}: is 1, 2 or 3;
   - X_{c}: is O or a direct bond;
   - R_{1c}: is H; C₁₋₆ alkyl optionally substituted by OH, acyl, halogen, C₃₋₁₀cycloalkyl, phenyl or hydroxy-phenylene; C₂₋₆alkenyl; C₂₋₆alkynyl; or phenyl optionally substituted by OH;
   - R_{2c}: is 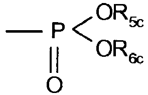
   wherein R_{5c} is H or C₁₋₄alkyl optionally substituted by 1, 2 or 3 halogen atoms, and R_{6c} is H or C₁₋₄alkyl optionally substituted by halogen;
   each of R_{3c} and R_{4c}, independently, is H, C₁₋₄alkyl optionally substituted by halogen, or acyl, and
   - R_{c}: is C₁₃₋₂₀alkyl which may optionally have in the chain an oxygen atom and which may optionally be substituted by nitro, halogen, amino, hydroxy or carboxy; or a residue of formula (a) 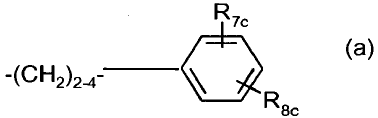 wherein R_{7c} is H, C₁₋₄alkyl or C₁₋₄alkoxy, and R_{8c} is substituted C₁₋₂₀alkanoyl, phenylC₁₋₁₄alkyl wherein the C₁₋₁₄alkyl is optionally substituted by halogen or OH, cycloalkylC₁₋₁₄alkoxy or phenylC₁₋₁₄alkoxy wherein the cycloalkyl or phenyl ring is optionally substituted by halogen, C₁₋₄alkyl and/or C₁₋₄alkoxy, phenylC₁₋₁₄alkoxy-C₁₋₁₄alkyl, phenoxyC₁₋₁₄alkoxy or phenoxyC₁₋₁₄alkyl,

   - R_{c}: being also a residue of formula (a) wherein R_{8c} is C₁₋₁₄alkoxy when R_{1c} is C₁₋₄alkyl, C₂₋₆alkenyl or C₂₋₆alkynyl,
   or a reference compound of formula VI 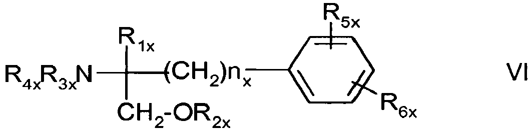 wherein
   - nₓ: is 2, 3 or 4
   - R₁ₓ: is H; C₁₋₆alkyl optionally substituted by OH, acyl, halogen, cycloalkyl, phenyl or hydroxy-phenylene; C₂₋₆alkenyl; C₂₋₆alkynyl; or phenyl optionally substituted by OH;
   - R₂ₓ: is H, C₁₋₄alkyl or acyl each of R₃ₓ and R₄ₓ, independently is H, C₁₋₄alkyl optionally substituted by halogen or acyl,
   - R₅ₓ: is H, C₁₋₄alkyl or C₁₋₄alkoxy, and
   - R₆ₓ: is C₁₋₂₀ alkanoyl substituted by cycloalkyl; cyloalkylC₁₋₁₄alkoxy wherein the cycloalkyl ring is optionally substituted by halogen, C₁₋₄alkyl and/or C₁₋₄alkoxy; phenylC₁₋₁₄alkoxy wherein the phenyl ring is optionally substituted by halogen, C₁₋₄alkyl and/or C₁₋₄alkoxy,
   - R₆ₓ: being also C₄₋₁₄alkoxy when R₁ₓ is C₂₋₄alkyl substituted by OH, or pentyloxy or hexyloxy when R₁ₓ is C₁₋₄akyl,
provided that R₆ₓ is other than phenyl-butylenoxy when either R₅ₓ is H or R₁ₓ is methyl, or a pharmaceutically acceptable salt thereof;
- Reference compounds as disclosed in WO02/06268Al, e.g. a compound of formula VII 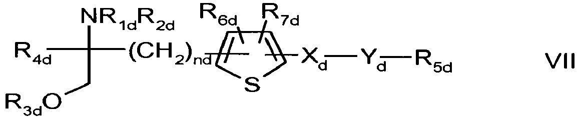 wherein each of R_{1d} and R_{2d}, independently, is H or an amino-protecting group; R_{3d} is hydrogen, a hydroxy-protecting group or a residue of formula 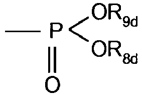 R_{4d} is lower alkyl;
   n_{d} is an integer of 1 to 6;
   X_{d} is ethylene, vinylene, ethynylene, a group having a formula - D-CH₂- (wherein D is carbonyl, - CH(OH)-, O, S or N), aryl or aryl substituted by up to three substitutents selected from group a as defined hereinafter;
   Y_{d} is single bond, C₁₋₁₀alkylene, C₁₋₁₀alkylene which is substituted by up to three substitutents selected from groups a and b, C₁₋₁₀alkylene having O or S in the middle or end of the carbon chain, or C₁₋₁₀alkylene having O or S in the middle or end of the carbon chain which is substituted by up to three substituents selected from groups a and b;
   R_{5d} is hydrogen, cycloalkyl, aryl, heterocycle, cycloalkyl substituted by up to three substituents selected from groups a and b, aryl substituted by up to three substituents selected from groups a and b, or heterocycle substituted by up to three substituents selected from groups a and b;
   each of R_{6d} and R_{7d}, independently, is H or a substituent selected from group a; each of R_{8d} and R_{9d}, independently, is H or C₁₋₄alkyl optionally substituted by halogen;
   <group a > is halogen, lower alkyl, halogeno lower alkyl, lower alkoxy, lower alkylthio, carboxyl, lower alkoxycarbonyl, hydroxy, lower aliphatic acyl, amino, mono-lower alkylamino, di-lower alkylamino, lower aliphatic acylamino, cyano or nitro; and
   <group b > is cycloalkyl, aryl, heterocycle, each being optionally substituted by up to three substituents selected from group a;
   with the proviso that when R_{5d} is hydrogen, Y_{d} is a either a single bond or linear C₁₋₁₀ alkylene, or a pharmacologically acceptable salt or ester thereof;
- Reference compounds as disclosed in JP-14316985 (JP2002316985), e.g. a compound of formula VIII 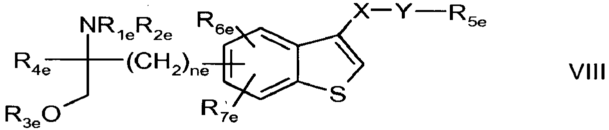 wherein R₁ₑ,R₂ₑ,R₃ₑ,R₄ₑ,R₅ₑ,R₆ₑ,R₇ₑ, nₑ, Xₑ and Yₑ are as disclosed in JP-14316985; or a pharmacologically acceptable salt or ester thereof;
- Reference compounds as disclosed in WO 03/29184 and WO 03/29205, e.g. compounds of formula IX 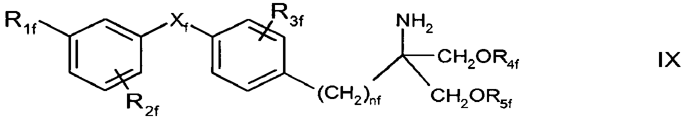 wherein X_{f} is O or S, and R_{1f}, R_{2f}, R_{3f} and n_{f} are as disclosed in WO 03/29184 and WO 03/29205, each of R_{4f} and R_{5f}, independently is H or a residue of formula 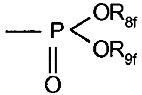 wherein each of R_{8f} and R_{9f}, independently, is H or C₁₋₄alkyl optionally substituted by halogen; e.g. 2-amino-2-[4-(3-benzyloxyphenoxy)-2-chlorophenyl]propyl-1,3-propane-diol or 2-amino-2-[4-(benzyloxyphenylthio)-2- chlorophenyl]propyl-1,3-propane-diol, or a pharmacological salt thereof;
- Reference compounds as disclosed in WO03/062252A1, e.g. a compound of formula X 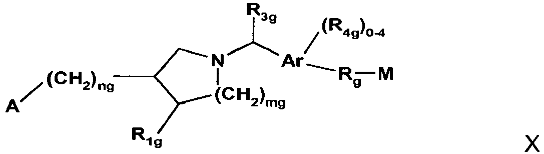 wherein
   Ar is phenyl or naphthyl; each of m_{g} and n_{g} independently is 0 or 1; A is selected from COOH, PO₃H₂, PO₂H, SO₃H, PO(C₁₋₃alkyl)OH and 1H-tetrazol-5-yl; each of R_{1g} and R_{2g} independently is H, halogen, OH, COOH or C₁₋₄alkyl optionally substituted by halogen; R_{3g} is H or C₁₋₄alkyl optionally substituted by halogen or OH; each R_{4g} independently is halogen, or optionally halogen substituted C₁₋₄alkyl or C₁₋₃alkoxy; and each of R_{g} and M has one of the significances as indicated for B and C, respectively, in WO03/062252A1;
- Compounds as disclosed in WO 03/062248A2, e.g. a compound of formula XI 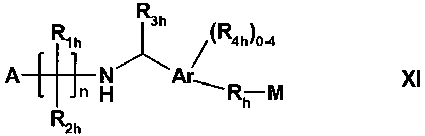 wherein Ar is phenyl or naphthyl; n is 2,3 or 4; A is COOH, 1*H*-tetrazol-5-yl, PO₃H₂, PO₂H₂, - SO₃H or PO(R₅ₕ)OH wherein R₅ₕ is selected from C₁₋₄alkyl, hydroxyC₁₋₄alkyl, phenyl, -CO-C₁₋₃alkoxy and -CH(OH)-phenyl wherein said phenyl or phenyl moiety is opitonally substituted; each of R₁ₕ and R₂ₕ independently is H, halogen, OH, COOH, or optionally halogeno substituted C₁₋₆alkyl or phenyl; R₃ₕ is H or C₁₋₄alkyl optionally substituted by halogen and/ OH; each R₄ₕ independently is halogeno, OH, COOH, C₁₋₄alkyl, S(O)_{0,1 or 2}C₁₋₃alkyl, C₁₋₃alkoxy, C₃₋₆cycloalkoxy, aryl or aralkoxy, wherein the alkyl portions may optionally be substituted by 1-3 halogens; and each of R_{g} and M has one of the significances as indicated for B and C, respectively, in WO03/062248A2.

When the compounds of formula I to XIII have one or more asymmetric centers in the molecule, the present disclosure is to be understood as embracing the various optical isomers, as well as racemates, diastereoisomers and mixtures thereof are embraced. Compounds of formula III or IVb, when the carbon atom bearing the amino group is asymmetric, have preferably the R-configuration at this carbon atom.

The compounds of formula I to XIII may exist in free or salt form. Examples of pharmaceutically acceptable salts of the compounds of the formula I to XIII include salts with inorganic acids, such as hydrochloride, hydrobromide and sulfate, salts with organic acids, such as acetate, fumarate, maleate, benzoate, citrate, malate, methanesulfonate and benzenesulfonate salts, or, when appropriate, salts with metals such as sodium, potassium, calcium and aluminium, salts with amines, such as triethylamine and salts with dibasic amino acids, such as lysine. The compounds and salts encompass hydrate and solvate forms.

Acyl as indicated above may be a residue R_{y}-CO- wherein Ry is C₁₋₆alkyl, C₃₋₆cycloalkyl, phenyl or phenyl-C₁₋₄alkyl. Unless otherwise stated, alkyl, alkoxy, alkenyl or alkynyl may be straight or branched.

When in the compounds of formula I the carbon chain as R₁ is substituted, it is preferably substituted by halogen, nitro, amino, hydroxy or carboxy. When the carbon chain is interrupted by an optionally substituted phenylene, the carbon chain is preferably unsubstituted. When the phenylene moiety is substituted, it is preferably substituted by halogen, nitro, amino, methoxy, hydroxy or carboxy.

Preferred reference compounds of formula I are those wherein R₁ is C₁₃₋₂₀alkyl, optionally substituted by nitro, halogen, amino, hydroxy or carboxy, and, more preferably those wherein R₁ is phenylalkyl substituted by C₆₋₁₄-alkyl chain optionally substituted by halogen and the alkyl moiety is a C₁₋₆alkyl optionally substituted by hydroxy. More preferably, R₁ is phenyl-C₁₋₆alkyl substituted on the phenyl by a straight or branched, preferably straight, C₆₋₁₄alkyl chain. The C₆₋₁₄alkyl chain may be in ortho, meta or para, preferably in para.

Preferably each of R₂ to R₅ is H.

A preferred reference compound of formula I is 2-amino-2-tetradecyl-1,3-propanediol. A particularly preferred compound in the embodiments of the invention of formula I is FTY720, i.e. 2-amino-2-[2-(4-octylphenyl) ethyl]propane-1,3-diol in free form or in a pharmaceutically acceptable salt form, e.g. the hydrochloride, as shown:

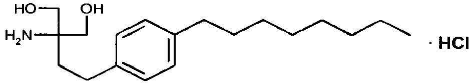

A preferred reference compound of formula II is the one wherein each of R'₂ to R'₅ is H and m is 4, i.e. 2-amino-2-{2-[4-(1-oxo-5-phenylpentyl)phenyl]ethyl}propane-1,3-diol, in free form or in pharmaceutically acceptable salt form (referred to hereinafter as Compound A), e.g the hydrochloride.

A preferred reference compound of formula III is the one wherein W is CH₃, each of R"₁ to R"₃ is H, Z₂ is ethylene, X is heptyloxy and Y is H, i.e. 2-amino-4-(4-heptyloxyphenyl)-2-methyl-butanol, in free form or in pharmaceutically acceptable salt form (referred to hereinafter as Compound B), e.g. the hydrochloride. The R-enantiomer is particularly preferred.

A preferred reference compound of formula IVa is the FTY720-phosphate (R₂ₐ is H, R₃ₐ is OH, Xₐ is O, R₁ₐ and R_{1b} are OH). A preferred compound of formula IVb is the Compound B-phosphate (R₂ₐ is H, R_{3b} is OH, Xₐ is O, R₁ₐ and R_{1b} are OH, Yₐ is O and R₄ₐ is heptyl). A preferred compound of formula V is Compound A-phosphate.

A preferred reference compound of formula V is phosphoric acid mono-[(R)-2-amino-2-methyl-4-(4-pentyloxy-phenyl)-butyl]ester.

A preferred reference compound of formula VIII is (2R)-2-amino-4-[3-(4-cyclohexyloxybutyl)-benzo[b]thien-6-yl]-2-methylbutan-1-ol.

In one aspect, a pharmaceutical organic concentrate formulation (hereinafter referred to as organic concentrate) is provided which comprises 2-amino-2-[2-(4-octylphenyl)ethyl]-propane-1,3-diol or a pharmaceutically acceptable salt thereof, in an organic solvent comprising 0-78.9 % (w/v) of ethanol (using a density value for ethanol of 0.789 gm/cm³) in propylene glycol. In a preferred embodiment, the organic solvent comprises 0-30 % (w/v) ethanol in propylene glycol. As an example, the organic solvent can comprise 20 % (w/v) ethanol in propylene glycol. As another example, the organic solvent can comprise 100 parts by volume of propylene glycol.

The compound 2-amino-2-[2-(4-octylphenyl)ethyl]-propane-1,3-diol or a pharmaceutically acceptable salt thereof can be prepared as described, e.g., in EP 0627406B1 and U.S. Patent No. 6,605,744. The preferred pharmaceutically acceptable salt of 2-amino-2-[2-(4-octylphenyl)ethyl]-propane-1,3-diol is hydrochloride. Examples of other pharmaceutically acceptable salts include, but are not limited to, hydrobromide, sulfate, acetate, fumarate, maleate, benzoate, citrate, malate, methanesulfonate, benzenesulfonate, and the like.

The afore-mentioned compound, 2-amino-2-[2-(4-octylphenyl)ethyl]-propane-1,3-diol or a pharmaceutically acceptable salt thereof is typically present in the organic solvent at a concentration of 0.01-10 mg/mL, preferably 0.1-5 mg/mL.

The organic concentrate can be prepared, e.g., by dissolving the afore-mentioned compound or a pharmaceutically acceptable salt thereof in either ethanol or propylene glycol separately, or when these solvents are utilized in combination, first dissolving the compound or a pharmaceutically acceptable salt thereof in ethanol followed by the addition of propylene glycol. As another example, when utilizing ethanol and propylene glycol in combination, the compound or a pharmaceutically acceptable salt thereof can be first dissolved in propylene glycol followed by the addition of ethanol. As another example, when utilizing ethanol and propylene glycol in combination, these two solvents can be mixed together followed by the addition of the compound or a pharmaceutically acceptable salt thereof. The organic concentrate can also include minor amounts of anti-oxidants, surfactants, buffers, preservatives, solubilizers, complexing agents, stabilizers, chelating agents, etc.

The organic concentrates are stable, i.e., free of crystals, for an extended period of time, e.g. at least six months at temperatures of 5°C or 25°C, as indicated in standard tests utilized to observe crystals in solutions (see Experimental Example).

The organic concentrates can be utilized as is in various topical or oral applications or other routes, or can be diluted prior to administration. Accordingly, in another aspect, a pharmaceutical solution is provided which comprises an organic concentrate as described above and a diluent vehicle.

The type of diluent vehicle selected for admixture with the organic concentrate will depend on the route of administration and in particular on whether the compound, according to the invention 2-amino-2-[2-(4-octylphenyl)ethyl]-propane-1,3-diol, is present in the organic concentrate in base or salt form. When the compound is in its salt form, the diluent vehicle typically comprises water or an isotonic solution such as a dextrose solution or a mannitol solution. When the compound is present as a free base, the diluent vehicle comprises an acidification agent, i.e., an inorganic or organic acid. Addition of an acidification agent to the organic concentrate containing the base form of the compound, prevents the base from precipitating out of the organic concentrate upon dilution with a water-based dilution vehicle by converting the base to a pharmaceutically acceptable salt. Examples of inorganic acids are hydrochloric acid, hydrobromic acid, sulfuric acid and the like. Examples of organic acids are acetic acid, fumaric acid, maleic acid, benzoic acid, citric acid, malic acid, methanesulfonic acid, benzenesulfonic acid, etc. The diluent vehicle utilized in admixture with the organic concentrate containing the base form of the compound can further comprise water, an isotonic solution, one or more solubilizers, e.g., surfactants, cyclodextrins and derivatives thereof, crystal inhibitors or combinations thereof.

The organic concentrate and diluent vehicle can be prepared and stored separately, e.g., as a pharmaceutical kit. Prior to administration the organic concentrate and diluent vehicle can be combined to form a pharmaceutical solution. The pharmaceutical solution so formed may be preferably used immediately or within a short time of being formed, e.g., within 24 hours. Alternatively, the organic concentrate and a predetermined amount of diluent, may be loaded each into separate chambers of a double-chamber vial system and only mixed immediately prior to administration, e.g., by i.v., to a patient.

The amount of diluent used in admixture with the organic concentrate to form a pharmaceutical solution may be chosen so as to obtain a desired concentration of one of the afore-mentioned compounds, according to the invention 2-amino-2-[2-(4-octylphenyl)ethyl]-propane-1,3-diol or a pharmaceutically acceptable salt thereof, in the pharmaceutical solution. The amount of diluent used is also chosen so that the solution is stable long enough to be administered. As an example, the concentration of pharmaceutical solutions made from the organic concentrates are typically 0.006 to 0.06 mg/mL. Such pharmaceutical solutions are found to be stable for up to 24 hours.

The pharmaceutical solution comprising the pharmaceutically acceptable salt of the compound can be prepared, e.g., by mixing the organic concentrate with a diluent, such as water or an isotonic solution, e.g., a dextrose solution or a mannitol solution, in a suitable container. The pharmaceutical solution comprising the base form of the compound, can be prepared, e.g., by first acidifying the organic concentrate with an acid, e.g., hydrochloride, to convert the base into a salt, followed by the addition of solubilizers, water, an isotonic solution, crystal inhibitors or combinations thereof. The pharmaceutical solution can further comprise minor amounts of antioxidants, surfactants, solubilizers, complexing agents, stabilizers, chelating agents, buffering agents, preservatives, moisturizing agents, pH adjusting agents, isotonizing agents or combinations thereof.

A preferred pharmaceutical solution of a salt form of 2-amino-2-[2-(4-octylphenyl)ethyl]-propane-1,3-diol comprises 1 mL of 1 mg/mL organic concentrate diluted with 99 mL of 5% dextrose to yield a solution containing 0.01 mg/mL.

In another aspect, a pharmaceutical semi-aqueous concentrate formulation (hereinafter referred to as a semi-aqueous concentrate) is provided which comprises a pharmaceutically acceptable salt of one of the afore-mentioned compounds, according to the invention 2-amino-2-[2-(4-octylphenyl)ethyl]-propane-1,3-diol, in a semi-aqueous solvent comprising 40-83% (w/v) of an organic component (using a density of 0.8301 gm/cm³ for propylene glycol to ethanol, 1:9 weight ratio) in water, wherein the organic component contains 10-90 parts by weight of ethanol and 10-90 parts by weight of propylene glycol having a combined total of 100. In a preferred embodiment, the semi-aqueous solvent comprises 50-70 % (w/v) of the organic component in water, wherein the organic component contains 10-30 parts by weight of ethanol and 70-90 parts by weight of propylene glycol. As an example, the semi-aqueous solvent comprises 50 % (w/v) of an organic component in water, wherein the organic component contains 20 parts by weight of ethanol and 80 parts by weight of propylene glycol.

The pharmaceutically acceptable salt of one of the afore-mentioned compounds, in the invention embodiments 2-amino-2-[2-(4-octylphenyl)ethyl]-propane-1,3-diol, can be present in the semi-aqueous solvent at a concentration of 0.01-5 mg/mL, and preferably at a concentration of 0.1-0.5 mg/mL.

The semi-aqueous concentrate can be prepared, e.g., by mixing ethanol with propylene glycol, dissolving the pharmaceutically acceptable salt of 2-amino-2-[2-(4-octylphenyl)ethyl]-propane-1,3-diol in the ethanol/propylene mixture, followed by the addition of deionized water.

The semi-aqueous concentrate can be utilized as is for certain applications, e.g., oral or topical or other routes, or can be diluted prior to its administration. Accordingly, in another aspect, a pharmaceutical solution is provided which comprises the afore-mentioned semi-aqueous concentrate and a diluent vehicle. The diluent vehicle typically comprises water, an isotonic solution, e.g., a dextrose solution or a mannitol solution, one or more solubilizers, e.g., surfactants, cyclodextrins or derivatives thereof, crystal inhibitors or combinations thereof.

The amount of diluent used in admixture with the semi-aqueous concentrate to form a pharmaceutical solution may be chosen so as to obtain a desired concentration of a pharmaceutically acceptable salt thereof in the pharmaceutical solution. The amount of diluent used is also chosen so that the pharmaceutical solution is stable long enough to be administered. As an example, the concentration of the pharmaceutical solutions prepared from a semi-aqueous concentrate are typically 0.006 to 0.06 mg/mL. Such pharmaceutical solutions are found to be stable for up to 24 hours.

The pharmaceutical solution made up of a semi-aqueous concentrate containing a pharmaceutically acceptable salt of one of the afore-mentioned compounds, in the invention embodiments 2-amino-2-[2-(4-octylphenyl)ethyl]-propane-1,3-diol hydrochloride, can be prepared, e.g., by mixing the semi-aqueous concentrate with a diluent, such as water or an isotonic solution in a suitable container. The semi-aqueous pharmaceutical formulations can also include minor amounts of antioxidants, surfactants, solubilizers, complexing agents, stabilizers, chelating agents, buffering agents, preservatives, moisturizing agents, pH adjusting agents and isotonizing agents.

A preferred pharmaceutical solution of a salt form of 2-amino-2-[2-(4-octylphenyl)ethyl]-propane-1,3-diol comprises 3 mL of 0.3 mg/mL semi-aqueous concentrate diluted with 97 mL of 5% dextrose to yield a solution containing 0.01 mg/mL.

The semi-aqueous concentrate and diluent vehicle can be prepared and stored separately, e.g, as a pharmaceutical kit. Prior to administration the semi-aqueous concentrate and diluent vehicle can be combined to form a pharmaceutical solution. The pharmaceutical solution so formed may be preferably used immediately or within a short time of being formed, e.g., up to 24 hours. Alternatively, the semi-aqueous concentrate and a predetermined amount of diluent, may be loaded each into separate chambers of a double-chamber vial system and only mixed immediately prior to administration, e.g., by i.v., to a patient.

The organic and semi-aqueous concentrates and their respective pharmaceutical solutions as described above can be administered by conventional routes such as oral, topical and parenteral, e.g., by infusion or injection. In a preferred embodiment, the pharmaceutical solutions prepared from either the organic concentrate or semi-aqueous concentrate, are administered parenterally as an infusion solution.

Accordingly, in one embodiment, for reference purposes, a method is provided for administering one of the afore-mentioned compounds, e.g., 2-amino-2-[2-(4-octylphenyl)ethyl]-propane-1,3-diol or a pharmaceutically acceptable salt thereof, for the treatment of a disease sensitive to treatment with the compound or a pharmaceutically acceptable salt thereof to a patient in need of such treatment, the method comprising administering the organic concentrate or semi-aqueous concentrate to the patient.

In another embodiment, for reference purposes, a method is provided for administering one of the afore-mentioned compounds, e.g., 2-amino-2-[2-(4-octylphenyl)ethyl]-propane-1,3-diol or a pharmaceutically acceptable salt thereof, for the treatment of a disease sensitive to treatment with the compound or a pharmaceutically acceptable salt thereof to a patient in need of such treatment, the method comprising:
(a) diluting the organic concentrate or semi-aqueous concentrate with a diluent vehicle to form a pharmaceutical solution; and
(b) administering the pharmaceutical solution to the patient.

The organic and semi-aqueous concentrates and pharmaceutical solutions of the present invention can be used for the suppression of rejection after organ or bone marrow transplantation, immunosuppressive sustention therapy, treatment of eye diseases such as Behcet's disease and uveitis, and dermatitis inclusive of psoriasis, atopic dermatitis, contact dermatitis and allergic dermatitis. In particular, the concentrates and pharmaceutical solutions can be used for the prophylaxis and treatment of various applicable diseases (e.g., immunosuppressant for organ or bone marrow transplantation, various autoimmune diseases, various allergic diseases and the like).

The organic and semi-aqueous concentrates and pharmaceutical solutions of the present invention can be used, in the treatment and prophylaxis of resistance or rejection in organ or tissue transplantation (e.g., transplantation of heart, kidney, liver, lung, bone marrow, cornea, pancreas, small intestine, limb, muscle, nerves, fatty marrow, duodenum, skin and pancreatic islet cell, and xeno-transplantation), graft-versus-host (GvH) diseases by bone marrow or small intestine transplantation, autoimmune diseases such as rheumatoid arthritis, systemic lupus erythematosus, nephrotic syndrome lupus, Hashimoto's thyroiditis, multiple sclerosis, myasthenia gravis, type I diabetes mellitus, type II adult onset diabetes mellitus, uveitis, nephrotic syndrome, steroid-dependent and steroid-resistant nephrosis, palmoplantar pustulosis, allergic encephalomyelitis, glomerulonephritis, etc., and infectious diseases caused by pathogenic microorganisms.

The organic and semi-aqueous concentrates and pharmaceutical solutions of the present invention are also useful for treating inflammatory, proliferative and hyperproliferative skin diseases and cutaneous manifestations of immunologically-mediated illnesses such as psoriasis, psoriatic arthritis, atopic eczema (atopic dermatitis), contact dermatitis and further eczematous dermatitises, seborrheic dermatitis, lichen planus, pemphigus, bullous pemphigoid, epidermolysis bullosa, urticaria, angioedemas, vasculitides, erythemas, cutaneous eosinophilias, acne, alopecia areata, eosinophilic fasciitis, and atherosclerosis. More particularly, the concentrates and pharmaceutical solutions of the present invention are useful in hair revitalizing, such as in the treatment of female or male pattern alopecia, or senile alopecia, by providing epilation prevention, hair germination, and/or a promotion of hair generation and hair growth.

The organic and semi-aqueous concentrates and pharmaceutical solutions of the present invention are further useful in the treatment of respiratory diseases, for example, sarcoidosis, fibroid lung, idiopathic interstitial pneumonia, and reversible obstructive airways disease, including conditions such as asthma, including bronchial asthma, infantile asthma, allergic asthma, intrinsic asthma, extrinsic asthma and dust asthma, particularly chronic or inveterate asthma (e.g., late asthma and airway hyperresponsiveness), bronchitis and the like. The organic and semi-aqueous concentrates and pharmaceutical solutions of the present invention may be also useful for treating hepatic injury associated with ischemia. The concentrates and pharmaceutical solutions of the present invention are also applied to certain eye diseases such as conjunctivitis, keratoconjunctivitis, keratitis, vernal conjunctivitis, uveitis associated with Behcet's disease, herpetic keratitis, conical cornea, dystorphia epithelialis corneae, keratoleukoma, ocular pemphigus, Mooren's ulcer, scleritis, Graves' ophthalmopathy, severe intraocular inflammation and the like.

The organic and semi-aqueous concentrates and pharmaceutical solutions of the present invention are also useful for preventing or treating inflammation of mucosa or blood vessels (e.g., leukotriene B4-mediated diseases, gastric ulcers, vascular damage caused by ischemic diseases and thrombosis, ischemic bowel disease, inflammatory bowel disease (e.g., Crohn's disease and ulcerative colitis), necrotizing enterocolitis), or intestinal lesions associated with thermal burns. The organic and semi-aqueous concentrates and pharmaceutical solutions of the present invention are further useful for treating or preventing renal diseases including interstitial nephritis, Goodpasture's syndrome, hemolytic uremic syndrome and diabetic nephropathy; nervous diseases selected from multiple myositis, Guillain-Barre syndrome, Meniere's disease and radiculopathy; endocrine diseases including hyperthyroidism and Basedow's disease; hematic diseases including pure red cell aplasia, aplastic anemia, hypoplastic anemia, idiopathic thrombocytopenic purpura, autoimmune hemolytic anemia, agranulocytosis and anerythroplasia; bone diseases including osteoporosis; respiratory diseases including sarcoidosis, fibroid lung and idiopathic interstitial pneumonia; skin diseases including dermatomyositis, vitiligo vulgaris, ichthyosis vulgaris, photoallergic sensitivity and cutaneous T cell lymphoma; circulatory diseases including arteriosclerosis, aortitis, polyarteritis nodos and amyocardosis; collagen disease including scieroderma, Wegener's granuloma and Sjogren's syndrome; adiposis;
eosinophilic fasciitis; periodontal disease; nephrotic syndrome; hemolytic uremic syndrome: and muscular dystrophy.

Further, the concentrates and pharmaceutical solutions of the present invention are indicated in the prophylaxis and treatment of diseases including intestinal inflammations or allergies such as Coeliac disease, proctitis, eosinophilic gastroenteritis, mastocytosis, Crohn's disease or ulcerative colitis; and food related allergic diseases which have symptomatic manifestation remote from the gastrointestinal tract, for example, migraine, rhinitis and eczema.

Generally, the organic concentrate, the semi-aqueous concentrate, and pharmaceutical solutions made therefrom, may be administered in an amount which is therapeutically effective against a disease or condition which can be treated by administration of 2-amino-2-[2-(4-octylphenyl)ethyl]-propane-1,3-diol or a pharmaceutically acceptable salt thereof, or one of the other disclosed compounds. The exact amount of 2-amino-2-[2-(4-octylphenyl)ethyl]-propane-1,3-diol or pharmaceutically acceptable salt thereof or other disclosed compound to administer can vary widely. However, the dose administered to an animal, particularly a human should be sufficient to effect a therapeutic response. The dose may depend on the particular compound, route of administration, the rate of administration, the strength of the particular concentrate or pharmaceutical solution employed, the nature of the disease or condition being treated, and the sex, age and body weight of the patient.. The size of the dose may also depend on the existence, nature and extent of any adverse side-effects that may accompany the administration of the concentrate or pharmaceutical formulation.

The organic and semi-aqueous concentrates and their respective pharmaceutical solutions can be used in combination with other immunosuppressant(s), steroid(s) (e.g., prednisolone, methylprednisolone, dexamethasone, hydrocortisone and the like) or nonsteroidal antiinflammatory agent. The administration of a combination of active agents may be simultaneous or consecutive, with either one of the active agents being administered first. The dosage of the active agents of a combination treatment may depend on effectiveness and site of action of each active agent, as well as synergistic effects between the agents used for combination therapy.

The present invention is described in more detail by referring to Examples and Comparative Examples. In the examples, the present compound refers to 2-amino-2-[2-(4-octylphenyl)ethyl]-propane-1,3-diol hydrochloride.

### Example 1

### 1.0 mg/mL of the present compound in 20/80 (^{w}/ᵥ) ethanol/propylene glycol

100 mg of the present compound is dissolved in 20 g of ethanol followed by the addition of propylene glycol to qs. 100 mL.

### Example 2

### 1.0 mg/mL of the present compound in 100% propylene glycol

100 mg of the present compound is dissolved in 100 mL propylene glycol.

### Example 3

### 0.3 mg/mL of the present compound in 10/40 (^{w}/_{w}) ethanol/propylene glycol in water

10 g of ethanol are mixed with 40 g of propylene glycol, followed by dissolving 0.3 mg of the present compound in the ethanol/propylene glycol mixture. Subsequent, deionized water or water for injection (WFI) is added to qs. 100 mL.

### Comparative Example 1

### 0.1-10 mg/mL of the present compound in water

0.1-10 mg of the present compound is dissolved in 100 mL of deionized water.

### Comparative Example 2

### 0.2 mg/mL of the present compound in 5% ethanol in water

0.2 mg of the present compound is dissolved 5 g of ethanol, followed by the addition of deionized water to qs. 100 mL.

### Experimental Example

### Physical stability

Solution clarity of Examples 1-3 and Comparative Examples 1 and 2 is monitored by manual inspection hood and microscopy. Formulations are stored in 25°C and 5°C chambers. The samplings are performed at two weeks, one, three and six months time points. At the pre-determined time, samples are pulled and examined for clarity and the absence of crystals as described below.

The clarity of the solution samples are primarily checked by the manual particulate inspection hood (M.W. Technologies Inc.). Each sample (in a clear vial) is placed against black background under Tyndal light. The vial is gently swirled in a circular motion. Subvisible crystals are easily observed as they move and reflect the light.

The microscopic method is performed to confirm the shape and size of the crystals. The solution is sampled and dropped onto a clean glass slide and the presence or absence of crystals is observed under the microscope.

Utilizing the manual particulate inspection hood and microscopic methods, all the samples taken from solutions prepared in Examples 1-3 are observed to be clear with no visible crystals. Samples taken from solutions prepared in Comparative Examples 1 and 2 are hazy and found to contain crystals.

## Claims

1. A pharmaceutical organic concentrate formulation comprising 2-amino-2-[2-(4-octylphenyl)ethyl]-propane-1,3-diol, or a pharmaceutically acceptable salt thereof, at a concentration of 0.01-10 mg/ml in an organic solvent comprising 0-78.9% (w/v) of ethanol in propylene glycol.

2. The organic concentrate formulation of Claim 1, wherein the organic solvent comprises 0-30% (w/v) ethanol in propylene glycol.

3. The organic concentrate formulation of Claim 1, wherein the organic solvent comprises 20% (w/v) ethanol in propylene glycol.

4. The organic concentrate formulation of Claim 1, wherein the organic solvent comprises 100 parts by volume of propylene glycol.

5. The organic concentrate organic formulation of any one of Claims 1 to 4 , wherein the compound, or salt thereof is at a concentration of 0.1-5 mg/mL.

6. The organic concentrate formulation of any preceding claim comprising 2-amino-2-[2-(4-octylphenyl)ethyl]-propane-1,3-diol hydrochloride as the pharmaceutically acceptable salt of 2-amino-2-[2-(4-octylphenyl)ethyl]-propane-1,3-diol.

7. A pharmaceutical solution comprising a pharmaceutical organic concentrate formulation according to any preceding Claims and a diluent vehicle, wherein the diluent vehicle optionally comprises an acidification agent.

8. The pharmaceutical solution of Claim 7, wherein the diluent vehicle further comprises water, an isotonic solution, a solubilizer, a crystal inhibitor or combinations thereof.

9. The pharmaceutical solution of Claim 8, wherein the concentrate formulation comprises 0.1-5 mg/mL of a pharmaceutically acceptable salt of the compound in 0-30 parts by weight of ethanol and 70-100 parts by weight of propylene glycol; and wherein the diluent vehicle comprises an isotonic solution.

10. A pharmaceutical kit comprising a concentrate formulation of any one of Claims 1 to 6 and a diluent vehicle, wherein the concentrate formulation and diluent vehicle are optionally in separate containers.

11. A method for preparing a pharmaceutical formulation as mentioned in claim 8 for administration comprising:
(a) preparing a solution of a 2-amino-1,3-propanediol compound, or a pharmaceutically acceptable salt thereof in an organic solvent comprising 0-78.9% (w/v) of ethanol in propylene glycol; and
(b) diluting the solution prepared in (a) with a diluent vehicle, wherein the 2 amino-1,3-propanediol compound is 2-amino-2-[2-(4-octylphenyl)ethyl]-propane-1,3-diol.

12. The method of claim 11, wherein the compound is 2-amino-2-[2-(4-octylphenyl)ethyl]-propane-1,3-diol hydrochloride.

13. A pharmaceutical semi-aqueous concentrate formulation according to claim 1 comprising a pharmaceutically acceptable salt of a 2-amino-1,3-propaned ol compound, in a semi-aqueous solvent comprising 40-83% (w/v) of an organic component in water, wherein the organic component contains 10-90 parts by weight of ethanol and 10-90 parts by weight of propylene glycol having a combined total of 100, wherein the pharmaceutically acceptable salt of the 2-amino-1,3-propane diol compound is 2-amino-2-[2-(4-octylphenyl)ethyl]-propane-1,3-diol hydrochloride.

14. The semi-aqueous concentrate formulation of Claim 13, wherein the semi-aqueous solvent comprises 50-70 % (w/v) of an organic component in water, and wherein the organic component contains 10-30 parts by weight of ethanol and 70-90 parts by weight of propylene glycol.

15. The semi-aqueous concentrate formulation of Claim 13, wherein the organic component contains 20 parts by weight of ethanol and 80 parts by weight of propylene glycol.

16. The semi-aqueous concentrate formulation of any one of Claims 13 to 15 wherein the salt of the 2-amino-1,3-propanediol compound is at a concentration of 0.01-5 mg/mL.

17. The semi-aqueous concentrate formulation of any one of Claims 13 to 15, wherein the salt of the 2-amino-1,3-propanediol compound is at a concentration of 0.1-0.5 mg/mL.

18. A pharmaceutical kit comprising a concentrate formulation of any one of claims 13 to 17 and a diluent vehicle, wherein the concentrate formulation and diluent vehicle are optionally in separate containers.

## Patentansprüche

1. Pharmazeutische organische Konzentratformulierung, umfassend 2-Amino-2-[2-(4-octylphenyl)ethyl]-propan-1,3-diol oder ein pharmazeutisch annehmbares Salz davon in einer Konzentration von 0,01-10 mg/ml in einem organischen Lösungsmittel, das 0-78,9 % (Gew./Vol.) Ethanol in Propylenglykol umfasst.

2. Organische Konzentratformulierung nach Anspruch 1, wobei das organische Lösungsmittel 0-30 % (Gew./Vol.) Ethanol in Propylenglykol umfasst.

3. Organische Konzentratformulierung nach Anspruch 1, wobei das organische Lösungsmittel 20 % (Gew./Vol.) Ethanol in Propylenglykol umfasst.

4. Organische Konzentratformulierung nach Anspruch 1, wobei das organische Lösungsmittel 100 Volumenteile Propylenglykol umfasst.

5. Organische Konzentratformulierung nach einem der Ansprüche 1 bis 4, wobei die Verbindung oder das Salz davon in einer Konzentration von 0,1-5 mg/ml vorliegt.

6. Organische Konzentratformulierung nach einem der vorhergehenden Ansprüche, umfassend 2-Amino-2-[2-{4-octylphenyl)ethyl]-propan-1,3-diol-hydrochlorid als pharmazeutisch annehmbares Salz von 2-Amino-2-[2-(4-octylphenyl)ethyl]-propan-1,3-diol.

7. Pharmazeutische Lösung, umfassend eine pharmazeutische organische Konzentratformulierung nach einem der vorhergehenden Ansprüche und ein Verdünnungsvehikel, wobei das Verdünnungsvehikel gegebenenfalls ein Azidifizierungsmittel umfasst.

8. Pharmazeutische Lösung nach Anspruch 7, wobei das Verdünnungsvehikel ferner Wasser, eine isotone Lösung, ein Solubilisierungsmittel, einen Kristallinhibitor oder Kombinationen davon umfasst.

9. Pharmazeutische Lösung nach Anspruch 8, wobei die Konzentratformulierung 0,1-5 mg/ml eines pharmazeutisch annehmbaren Salzes der Verbindung in 0-30 Gewichtsteilen Ethanol und 70-100 Gewichtsteilen Propylenglykol umfasst; und wobei das Verdünnungsvehikel eine isotone Lösung umfasst.

10. Pharmazeutisches Kit, umfassend eine Konzentratformulierung nach einem der Ansprüche 1 bis 6 und ein Verdünnungsvehikel, wobei die Konzentratformulierung und das Verdünnungsvehikel optional in getrennten Behältern vorliegen.

11. Verfahren zum Herstellen einer pharmazeutischen Formulierung nach Anspruch 8 zur Verabreichung, umfassend:
(a) Herstellen einer Lösung einer 2-Amino-1,3-propandiolverbindung oder eines pharmazeutisch annehmbaren Salzes davon in einem organischen Lösungsmittel, umfassend 0-78,9 % (Gew./Vol.) Ethanol in Propylenglykol; und
(b) Verdünnen der in (a) hergestellten Lösung mit einem Verdünnungsvehikel,
wobei die 2-Amino-1,3-propandiolverbindung 2-Amino-2-[2-(4-octylphenyl)-ethyl]-propan-1,3-diol ist.

12. Verfahren nach Anspruch 11, wobei die Verbindung 2-Amino-2-[2-(4-octyl-phenyl)ethyl]-propan-1,3-diol-hydrochlorid ist.

13. Pharmazeutische halbwässrige Konzentratformulierung nach Anspruch 1, umfassend ein pharmazeutisch annehmbares Salz einer 2-Amino-1,3-pro-pandiolverbindung in halbwässrigem Lösungsmittel, umfassend 40-83 % (Gew./Vol.) einer organischen Komponente in Wasser, wobei die organische Komponente 10-90 Gewichtsteile Ethanol und 10-90 Gewichtsteile Propylenglykol mit einer kombinierten Summe von 100 enthält, wobei das pharmazeutisch annehmbare Salz der 2-Amino-1,3-propandiolverbindung 2-Amino-2-[2-(4-octylphenyl)ethyl]-propan-1,3-diol-hydrochlorid ist.

14. Halbwässrige Konzentratformulierung nach Anspruch 13, wobei das halbwässrige Lösungsmittel 50-70 % (Gew./Vol.) einer organischen Komponente in Wasser umfasst, und wobei die organische Komponente 10-30 Gewichtsteile Ethanol und 70-90 Gewichtsteile Propylenglykol enthält.

15. Halbwässrige Konzentratformulierung nach Anspruch 13, wobei die organische Komponente 20 Gewichtsteile Ethanol und 80 Gewichtsteile Propylenglykol enthält.

16. Halbwässrige Konzentratformulierung nach einem der Ansprüche 13 bis 15, wobei das Salz der 2-Amino-1,3-propandiolverbindung in einer Konzentration von 0,01-5 mg/ml vorliegt.

17. Halbwässrige Konzentratformulierung nach einem der Ansprüche 13 bis 15, wobei das Salz der 2-Amino-1,3-propandiolverbindung in einer Konzentration von 0,1-0,5 mg/ml vorliegt.

18. Pharmazeutisches Kit, umfassend eine Konzentratformulierung nach einem der Ansprüche 13 bis 17 und ein Verdünnungsvehikel, wobei die Konzentratformulierung und das Verdünnungsvehikel optional in getrennten Behältern vorliegen.

## Revendications

1. Formulation de concentré organique pharmaceutique contenant du 2-amino-2-[2-(4-octylphényl)éthyl]-propane-1,3-diol, ou un sel pharmaceutiquement acceptable de celui-ci, à une concentration de 0,01 à 10 mg/ml dans un solvant organique contenant 0 à 78,9 % (en poids sur volume) d'éthanol dans du propylène glycol.

2. Formulation de concentré organique selon la revendication 1, dans laquelle le solvant organique comprend 0 à 30 % (en poids sur volume) d'éthanol dans du propylène glycol.

3. Formulation de concentré organique selon la revendication 1, dans laquelle le solvant organique comprend 20 % (p/v) d'éthanol dans du propylène glycol.

4. Formulation de concentré organique selon la revendication 1, dans laquelle le solvant organique comprend 100 parties en volume de propylène glycol.

5. Formulation de concentré organique selon l'une quelconque des revendications 1 à 4, dans laquelle le composé ou son sel est à une concentration de 0,1 à 5 mg/ml.

6. Formulation de concentré organique selon l'une quelconque des revendications précédentes, comprenant du chlorhydrate de 2-amino-2-[2-(4-octylphényl)éthyl]-propane-1,3-diol en tant que sel pharmaceutiquement acceptable du 2-amino-2-[2-(4-octylphényl)éthyl]-propane-1,3-diol.

7. Solution pharmaceutique contenant un concentré organique pharmaceutique selon l'une quelconque des revendications précédentes et un véhicule diluant, le véhicule diluant comprenant éventuellement un agent d'acidification.

8. Solution pharmaceutique selon la revendication 7, dans laquelle le véhicule diluant comprend en outre de l'eau, une solution isotonique, un solubilisant, un inhibiteur cristallin ou des combinaisons de ceux-ci.

9. Solution pharmaceutique selon la revendication 8, dans laquelle la formulation concentrée comprend 0,1 à 5 mg/ml d'un sel pharmaceutiquement acceptable du composé dans 0 à 30 parties en poids d'éthanol et 70 à 100 parties en poids de propylène glycol ; et où le véhicule diluant comprend une solution isotonique.

10. Kit pharmaceutique comprenant une formulation concentrée selon l'une quelconque des revendications 1 à 6 et un véhicule diluant, la formulation de concentré et le véhicule diluant étant éventuellement dans des récipients séparés.

11. Procédé de préparation d'une formulation pharmaceutique selon la revendication 8 pour administration, comprenant :
(a) la préparation d'une solution d'un composé de 2-amino-1,3-propanediol ou d'un sel pharmaceutiquement acceptable de celui-ci dans un solvant organique contenant 0 à 78,9 % (p/v) d'éthanol dans du propylène glycol ; et
(b) la dilution de la solution préparée en (a) avec un véhicule diluant, le composé 2-amino-1,3-propanediol étant le 2-amino-2-[2-(4-octylphényl)éthyl]-propane-1,3-diol.

12. Procédé selon la revendication 11, dans lequel le composé est le chlorhydrate de 2-amino-2-[2-(4-octylphényl)éthyl]-propane-1,3-diol.

13. Formulation de concentré semi-aqueux pharmaceutique selon la revendication 1, contenant un sel pharmaceutiquement acceptable d'un composé de 2-amino-1,3-propanediol dans un solvant semi-aqueux contenant 40 à 83 % (en poids sur volume) d'un composant organique dans de l'eau, le composant organique contenant 10 à 90 parties en poids d'éthanol et 10 à 90 parties en poids de propylène glycol comptant un total combiné de 100, le sel pharmaceutiquement acceptable du composé de 2-amino-1,3-propanediol étant le chlorhydrate de 2-amino-2-[2-(4-octylphényl)éthyl]-propane-1,3-diol.

14. Formulation de concentré semi-aqueux selon la revendication 13, où le solvant semi-aqueux contient 50 à 70 % (p/v) d'un composant organique dans de l'eau, et où le composant organique contient 10 à 30 parties en poids d'éthanol et 70 à 90 parties en poids de propylène glycol.

15. Formulation de concentré semi-aqueux selon la revendication 13, où le composant organique contient 20 parties en poids d'éthanol et 80 parties en poids de propylène glycol.

16. Formulation de concentré semi-aqueux selon l'une quelconque des revendications 13 à 15, où le sel du composé de 2-amino-1,3-propanediol est à une concentration de 0,01 à 5 mg/ml.

17. Formulation de concentré semi-aqueux selon l'une quelconque des revendications 13 à 15, où le sel du composé de 2-amino-1,3-propanediol est à une concentration de 0,1 à 0,5 mg/mL.

18. Kit pharmaceutique comprenant une formulation concentrée selon l'une quelconque des revendications 13 à 17 et un véhicule diluant, la formulation de concentré et le véhicule diluant étant éventuellement dans des récipients séparés.
